Europäisches Patentamt

European Patent Office

Office européen des brevets

(19)

(11) **EP 0 461 473 B1**

(12) **EUROPÄISCHE PATENTSCHRIFT**

(45) Veröffentlichungstag und Bekanntmachung des Hinweises auf die Patenterteilung:
**27.03.1996 Patentblatt 1996/13**

(51) Int Cl.6: **G01N 33/92**, C12Q 1/61
// G01N33/84, C12Q1/60,
C12Q1/00, G01N33/50

(21) Anmeldenummer: **91108765.8**

(22) Anmeldetag: **29.05.1991**

(54) **Verfahren und Mittel zur Beseitigung von Trübungen in biologischen Flüssigkeiten**

Method and means for eliminating clouding in biological fluids

Procédé et moyen pour l'élimination d'opacité dans des fluides biologiques

(84) Benannte Vertragsstaaten:
**BE CH DE ES FR GB IT LI NL SE**

(30) Priorität: **09.06.1990 DE 4018502**

(43) Veröffentlichungstag der Anmeldung:
**18.12.1991 Patentblatt 1991/51**

(73) Patentinhaber: **MERCK PATENT GmbH**
**D-64271 Darmstadt (DE)**

(72) Erfinder: **Vormbrock, Rolf, Dr.**
**W-6100 Darmstadt (DE)**

(56) Entgegenhaltungen:
**EP-A- 0 004 857          EP-A- 0 009 596**
**EP-A- 0 130 537          EP-A- 0 141 879**

## Beschreibung

Die Erfindung betrifft ein Verfahren und Mittel zur Beseitigung von Trübungen in biologischen Flüssigkeiten unter Verwendung oberflächenaktiver Substanzen.

Bei der Analyse biologischer Flüssigkeiten stellen Trübungen, die durch den Gehalt der Probe an lipophilen Substanzen hervorgerufen werden, oftmals ein Problem für die photometrische Messung von Bestandteilen der Probe dar.

Bei der klinisch chemischen Untersuchung von Serum oder Plasma wurde daher auf verschiedene Weise versucht, diese Störung zu eliminieren. Bekannte Verfahren sind zum Beispiel die Extraktion der Probe mit einer mit Wasser nicht mischbaren organischen Flüssigkeit, die Ausfällung der in der Probe enthaltenen Lipidpartikel oder der Solubilisierung der Lipide. Extraktions- und Fällungsmethode erfordern einen Probenvorbereitungsschritt und führen zu Volumenveränderung der Probe, so daß diese Techniken für eine zuverlässige Routineanalytik nicht geeignet sind.

Zur Solubilisierung der Lipide sind Tensidmischungen verwendet worden, deren Zusammensetzung und Wirkung in der EP-PS 130 537 ausführlich beschrieben sind. In den meisten Fällen ist ab einer bestimmten Triglyceridkonzentration und bei Überschreitung eines bestimmten Probe/Reagenz-Verhältnisses die Wirksamkeit dieser Reagenzien nicht mehr gegeben, so daß die Trübung der Probe trotz der Zusätze im Reagenz zu falschen Meßwerten führt. Das in der EP-A-0 130 537 beschriebene Verfahren beruht auf der Kombination dreier Tenside und führte auch bei hoher Triglyceridkonzentration und hohem Probenanteil innerhalb von 10 Minuten zu einer Klärung der Probe.

In EP-A-0 004 857 ist ein Verfahren und Mittel zur Beseitigung von Trübungen in durch Probenmaterial getrübten Reaktionsgemischen durch Zusatz von Detergenzien beschrieben, wobei neben ein oder mehreren niedermolekularen organischen Verbindungen ein oder mehrere Detergenzien verwendet werden, die mit den niedermolekularen Verbindungen einen wenig löslichen Komplex bilden. Der ausgefällte Komplex wird dann durch die Zugabe von weiterem Detergenz wieder gelöst. Es hat sich gezeigt, daß ohne den Zusatz der niedermolekularen organischen Verbindungen trotz relativ hoher Detergenzkonzentrationen keine Klärung eintritt. Ein Nachteil ist auch in der Verwendung von z.B. chlorierten Phenolen, Chloranilin, Chlorofrom, Benzol usw. als niedermolekulare Verbindungen zu sehen.

Der Erfindung lag die Aufgabe zugrunde, ein Verfahren und Mittel zur Beseitigung von Trübungen in biologischen Flüssigkeiten zur Verfügung zu stellen, das auch bei einem hohen Probenanteil in kurzer Zeit eine vollständige Auflösung der Lipidpartikel bewirkt, ohne daß es zu Störungen der Nachweisreaktion kommt.

Gegenstand der Erfindung ist ein Verfahren zur Beseitigung von Trübungen in biologischen Flüssigkeiten durch Zusatz von oberflächenaktiven Mitteln, das dadurch gekennzeichnet ist, daß als oberflächenaktive Mittel

a. eine Alkyldimethylbenzylammoniumverbindung,
b. ein polyethoxylierter Triester des Sorbitans oder Sorbitols mit langkettigen Fettsäuren sowie gegebenenfalls
c. ein amphoteres Tensid

in wäßriger, gegebenenfalls gepufferter und salzhaltiger Lösung eingesetzt werden. Als amphoteres Tensid wird vozugsweise Lauroylsarkosin eingesetzt.

Ein weiterer Gegenstand der Erfindung ist ein Mittel zur Beseitigung von Trübungen in biologischen Flüssigkeiten, das dadurch gekennzeichnet ist, daß es

a. eine Alkyldimethylbenzylammoniumverbindung,
b. einen polyethoxylierten Triester des Sorbitans oder Sorbitols mit langkettigen Fettsäuren sowie gegebenenfalls
c. ein amphoteres Tensid

in wäßriger, gegebenenfalls gepufferter und salzhaltiger Lösung enthält. Als amphoteres Tensid enthält das Mittel vorzugsweise Lauroylsarkosin.

Überraschenderweise hat sich gezeigt, daß mit der erfindungsgemäßen Kombination oberflächenaktiver Mittel, selbst bei einem Probeanteil von 20 %, in deutlich weniger als 10 Minuten eine vollständige und dauerhafte Aufklarung erfolgt. Das erfindungsgemäße Reagenz ist dank des kationischen Tensids ohne zusätzliche Konservierungsmittel bei den für klinisch chemische Reagenzien üblichen Lagertemperaturen von 2-8 °C bzw. 15-25 °C stabil.

Als Alkyldimethylbenzylammoniumverbindungen eignen sich vorzugsweise Alkyldimethylbenzylammoniumchloride, wobei der Alkylrest, z. B. einen unverzweigten Kohlenwasserstoffrest mit einer Kettenlänge von 10 bis 14 C-Atomen darstellt. Solche Verbindungen sind z. B. unter den Markennamen Hyamine 3500® der Firma Lonza AG, Basel, oder Protectol KLC® der Firma BASF erhältlich. Ebenfalls geeignet sind Verbindungen, die verzweigte oder aromatische Kohlenwasserstoffketten und Etherbindungen in der Alkylkette tragen, z. B. Hyamine 1622® der Firma Lonza AG, Basel. Diese Verbindungen werden in wäßriger, gegebenenfalls gepufferter Lösung im pH-Bereich von 2 bis 12 in einer Konzentration von 0,5 bis 10 Gewichtsprozent eingesetzt.

Als polyethoxylierte Triester des Sorbitans oder Sorbitols mit langkettigen Fettsäuren eignen sich Polyoxyethylen-20-sorbitantrioleat, z. B. Tween 85® der Firme ICI, oder Polyoxyethylen-20-sorbitoltrioleat, z. B. Witsorbox CO® der Firma Witco, oder Polyoxyethylen-20-sorbitantristearat, z. B. Tween 65® der Firma ICI. Diese Verbindungen werden in wäßriger, gegebenenfalls gepufferter Lösung, die mindestens eine der erfindungsgemäßen Alkyldimethylbenzylammoniumverbindungen enthält, im pH-Bereich von 2 bis 12 in einer Konzentration von 0,5 bis 10 Gewichtsprozent eingesetzt.

Das Mittel nach der Erfindung kann neben den genannten kationischen und nichtionischen Tensiden gegebenenfalls auch ein amphoteres Tensid enthalten. Als amphotere Tenside eignen sich z. B. Amide des Sarkosins mit Carbonsäuren mit einer Kettenlänge von 12 bis 14 C-Atomen, wie N-Lauroylsarkosin, z. B. Medialan LD® der Firma Hoechst. Diese Verbindungen werden in wäßriger, gegebenenfalls gepufferter Lösung, die mindestens eine der erfindungsgemäßen Alkyldimethylbenzylammoniumverbindungen und mindestens eine der erfindungsgemäßen nichtionischen Tenside enthält, im pH-Bereich von 2 bis 12 in einer Konzentration von 0,5 bis 7 Gewichtsprozent eingesetzt.

Die Aufklarung gelingt mit dem erfindunsgemäßen Mittel innerhalb des in der klinischen Chemie üblichen Temperaturintervalls von etwa 15-37 °C. Als Puffer können alle in der klinischen Chemie bekannten Puffer verwendet werden, die einen pH-Bereich von 2 bis 10 aufrechterhalten können, vorzugsweise Phosphatpuffer, Trispuffer, PIPES-Puffer, Glycin/HCl-Puffer usw. Die Pufferkonzentration sollte im Bereich von 10-300 mmol/l liegen, vorzugsweise im Bereich von 25-200 mmol/l.

Das erfindungsgemäße Mittel kann außerdem ein Salz zur Erhöhung der Ionenstärke enthalten, z. B. Natrium- oder Kaliumchlorid in etwa der gleichen Konzentration wie die Pufferkonzentration.

Je nach dem in der biologischen Flüssigkeit zu bestimmenden Analyt kann das Mittel weitere Substanzen zur Bestimmung dieses Analyts enthalten.

Beispiel 1

Reagenz zur Bestimmung von Eisen im Serum

| Pufferlösung: | | |
| --- | --- | --- |
| | Konzentration im Reagenz | |
| Glycin/HCl-Puffer, pH 2,9 | 200 | mmol/l |
| Guanidiniumchlorid | 4,5 | mol/l |
| Ascorbinsäure | 60 | mmol/l |
| Hyamine 3500® | 50 | g/l |
| Tween 85® | 40 | g/l |
| Thioharnstoff | 12 | mmol/l |
| Farbreagenz: | | |
| Glycin/HCl-Puffer, pH 4,8 3-(2-Pyridyl)-5,6-bis(4-phenylsulfonsäure)-1,2,4-triazin, diNatriumsalz | 200 4,8 | mmol/l mmol/l |

Durchführung der Bestimmung:

| Temperatur: | 25 °C |
| --- | --- |
| Wellenlänge: | 562 nm |
| Schichtdicke: | 1 cm |

In eine Küvette werden pipettiert:

| | Analyse | Reagenzleerwert |
| --- | --- | --- |
| Pufferlösung | 2,0 ml | 2,0 ml |
| Probe | 0,4 ml | |
| bidestilliertes Wasser | | 0,4 ml |

Die Lösung wird gemischt, und nach 5 Minuten wird die Extinktion $E_1$ gemessen, danach wird zugegeben

| Startreagenz | 0,01 ml | 0,01 ml |
|---|---|---|

nach 3 Minuten wird die Extinktion $E_2$ gemessen.

Die Extinktionsdifferenz für Analyse $E_A = E_2 - E_1$ und Reagenzienleerwert $E_L = E_2 - E_1$ wird berechnet, und aus der Differenz $\Delta E = E_A - E_L$ wird die Eisenkonzentration nach folgender Gleichung berechnet:

$$c = 1195 \times \Delta E \ \mu g/dl$$

Während bei der Durchführung der Bestimmung ohne die erfindungsgemäßen Tenside die Trübung lipämischer Seren zu falschen Ergebnissen führt, was auch nicht durch die Messung eines Probenleerwertes kompensiert werden kann, ist bei Verwendung des erfindungsgemäßen Verfahrens, abhängig von der eingesetzten Tensidkonzentration, diese Störung bis zu Triglyceridkonzentrationen von 1000 mg/dl oder darüber eliminiert.

Beispiel 2

Reagenz zur Bestimmung von Glucose im Serum

Pufferlösung:

|  | Konzentration im Reagenz | |
|---|---|---|
| PIPES - Puffer, pH 7,2 | 50 | mmol/l |
| 1-Phenyl-2,3-dimethyl-4-amino-pyrazolon-(5) | 1 | mmol/l |
| Hydroxybenzoesäure | 6 | mmol/l |
| Hyamine 3500® | 25 | g/l |
| Tween 85® | 25 | g/l |
| N-Lauroylsarkosin | 5 | g/l |
| Glucoseoxidase | 6 | kU/l |
| Peroxidase | 3,2 | kU/l |
| Mutarotase | 0,05 | kU/l |

Durchführung der Bestimmung:

| Temperatur: | 25 °C |
|---|---|
| Wellenlänge: | 500 nm |
| Schichtdicke: | 1 cm |

In eine Küvette werden pipettiert:

|  | Analyse | Reagenzleerwert |
|---|---|---|
| Pufferlösung | 2,0 ml | 2,0 ml |
| Probe | 0,02 ml |  |
| bidestilliertes Wasser |  | 0,02 ml |

Die Lösung wird gemischt, und nach 5 Minuten wird die Extinktion gemessen.

Aus der Extinktionsdifferenz von Analyse und Reagenzleerwert $\Delta E = E_A - E_L$ wird die Glucosekonzentration nach folgender Gleichung berechnet:

$$c = 342 \times \Delta E \text{ mg/dl}$$

Während bei der Durchführung der Bestimmung ohne die erfindungsgemäßen Tenside die Trübung lipämischer Seren zu falschen Ergebnissen führt, ist bei Verwendung des erfindungsgemäßen Verfahrens, abhängig von der eingesetzten Tensidkonzentration, diese Störung bis zu Triglyceridkonzentrationen von 1000 mg/dl oder darüber eliminiert.

**Patentansprüche**

1. Verfahren zur Beseitigung von Trübungen in biologischen Flüssigkeiten durch Zusatz von oberflächenaktiven Mitteln, dadurch gekennzeichnet, daß als oberflächenaktive Mittel

   a. eine Alkyldimethylbenzylammoniumverbindung,
   b. ein polyethoxylierter Triester des Sorbitans oder Sorbitols mit langkettigen Fettsäuren sowie gegebenenfalls
   c. ein amphoteres Tensid

   in wäßriger, gegebenenfalls gepufferter und salzhaltiger Lösung eingesetzt werden.

2. Verfahren nach Anspruch 1, dadurch gekennzeichnet, daß als amphoteres Tensid Lauroylsarkosin eingesetzt wird.

3. Mittel zur Beseitigung von Trübungen in biologischen Flüssigkeiten, dadurch gekennzeichnet, daß es

   a. eine Alkyldimethylbenzylammoniumverbindung,
   b. einen polyethoxylierten Triester des Sorbitans oder sorbitols mit langkettigen Fettsäuren sowie gegebenenfalls
   c. ein amphoteres Tensid

   in wäßriger, gegebenenfalls gepufferter und salzhaltiger Lösung enthält.

4. Mittel nach Anspruch 3, dadurch gekennzeichnet, daß es als amphoteres Tensid Lauroylsarkosin enthält.

**Claims**

1. A process for the removal of turbidity from biological fluids by the addition of surface-active agents, characterised in that the surface-active agents used are

   a. an alkyldimethylbenzylammonium compound,
   b. a polyethoxylated triester of sorbitan or sorbitol with long-chain fatty acids, and, if appropriate,
   c. an amphoteric surfactant,

   in aqueous, optionally buffered and salt containing solution.

2. A process according to Claim 1, characterised in that lauroylsarcosine is used as the amphoteric surfactant.

3. A composition for the removal of turbidity from biological fluids, characterised in that it contains

   a. an alkyldimethylbenzylammonium compound,
   b. a polyethoxylated triester of sorbitan or sorbitol with long-chain fatty acids, and, if appropriate,
   c. an amphoteric surfactant,

   in aqueous, optionally buffered and salt containing solution.

4. A composition according to Claim 3, characterised in that it contains lauroylsarcosine as the amphoteric surfactant.

**Revendications**

1. Procédé pour éliminer les troubles dans les liquides physiologiques, par addition d'agents tensio-actifs, caractérisé en ce qu'on utilise comme agents tensioactifs

   a. un composé d'un alkyldiméthylbenzylammonium,
   b. un triester polyéthoxylé du sorbitan ou du sorbitol et d'acides gras à longue chaîne, et éventuellement
   c. un tensioactif amphotère,

   en solution aqueuse, éventuellement tamponnée et salée.

2. Procédé selon la revendication 1, caractérisé en ce qu'on utilise comme tensioactif amphotère la lauroyl-sarcosine.

3. Milieu pour éliminer les troubles dans les liquides biologiques, caractérisé en ce qu'il contient

   a. un composé d'un alkyldiméthylbenzylammonium,
   b. un triester polyéthoxylé du sorbitan ou du sorbitol et d'acides gras à longue chaîne, et éventuellement
   c. un tensioactif amphotère,

   en solution aqueuse, éventuellement tamponnée et salée.

4. Milieu selon la revendication 3, caractérisé en ce qu'il contient comme tensioactif amphotère la lauroyl-sarcosine.